(19) Europäisches Patentamt
European Patent Office
Office européen des brevets

(11) Veröffentlichungsnummer: **0 288 432 A1**

# (12) EUROPÄISCHE PATENTANMELDUNG

(21) Anmeldenummer: **88810214.2**

(22) Anmeldetag: **30.03.88**

(51) Int. Cl.[4]: **A 01 N 43/82**
C 07 D 417/14

(30) Priorität: **09.04.87 CH 1360/87 15.02.88 CH 543/88**

(43) Veröffentlichungstag der Anmeldung: **26.10.88 Patentblatt 88/43**

(84) Benannte Vertragsstaaten: **AT BE CH DE ES FR GB IT LI NL**

(71) Anmelder: **CIBA-GEIGY AG
Klybeckstrasse 141
CH-4002 Basel (CH)**

(72) Erfinder: **Farooq, Saleem, Dr.
Kirchackerstrasse 27
CH-4422 Arisdorf (CH)**

## (54) Schädlingsbekämpfungsmittel.

(57) Verwendung von 2,5-Bis-pyridyl-1,3,4-thiadiazolen der Formel

sowie ihrer Salze zur Bekämpfung von Schädlingen, insbesondere zur Bekämpfung von Pflanzen und Tiere befallenden Insekten. Die Verbindungen der Formel I weisen insbesondere gute Wirksamkeit gegen pflanzenschädigende saugende Insekten auf.

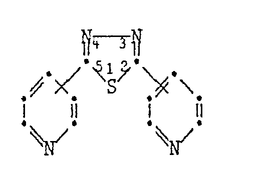

EP 0 288 432 A1

**Beschreibung**

**Schädlingsbekämpfungsmittel**

Die vorliegende Erfindung schlägt die Verwendung von 2,5-Bis-pyridyl-1,3,4-thiadiazolen zur Bekämpfung von Schädlingen sowie neuartige Salze und neuartige Verbindungen dieses Strukturtyps vor.

Die Erfindung betrifft zunächst die Verwendung einer Verbindung der Formel I

(I),

sowie der Salze einer Verbindung der Formel I zur Bekämpfung von Schädlingen.

Erfindungsgemäss bevorzugt verwendet werden Verbindungen der Formel Ia,

(Ia).

Für die erfindungsgemässen Zwecke von besonderer Bedeutung ist die Verbindung der folgenden Formel

Bei den Salzen der Verbindungen der Formel I handelt es sich um neuartige Stoffe. Somit umfasst der Erfindungsgegenstand auch die Salze, insbesondere pflanzenphysiologisch unbedenkliche Salze, der Verbindungen der Formel I sowie deren Verwendung als Schädlingsbekämpfungsmittel. Als derartige Salze mit organischen und anorganischen Säuren kommen z.B. die folgenden in Betracht: Chloride, Bromide, Jodide, Sulfate, Hydrogensulfate, Chlorate, Perchlorate, Rhodanide, Nitrate, Phosphate, Hydrogenphosphate, Tetrafluorborate, Formiate, Acetate, Trichloracetate, Trifluoracetate, Phenylsulfonate, Oxalate, Malonate, Succinate, Malate, Tartrate oder Citrate.

Die Erfindung schlägt weiterhin die nachstehend in Beispiel 1 aufgeführten und unter die vorliegende Formel I fallenden, jedoch per se neuartigen Verbindungen Nr. 2, 3 und 4 vor, die hohe insektizide Wirksamkeit besitzen.

Pestizid wirksame 2-(3-Pyridyl)-1,3,4-thiadiazole und 5-(3-Pyridyl-1,2,4-thiadiazole sind aus der europäischen Patentanmeldung 0.116.515 bekannt. 1,3,4-Oxidiazole ähnlicher Struktur mit pestizider Wirkung sind Gegenstand der europäischen Patentanmeldung 0.097.126. Verbindungen der vorliegenden Formel I, bzw. Verbindungen entsprechender Struktur, sind bereits in den schweizerischen Patentschriften 411,906 und 426,848 beschrieben worden. Gemäss diesen Patentschriften sind die Verbindungen als UV-Schutzmittel, optische Aufheller oder als Farbstoff-Zwischenprodukte brauchbar. Demgegenüber wurde nun gefunden, dass die zur erfindungsgemässen Verwendung vorgeschlagenen Verbindungen der vorliegenden Formel I bei guter Pflanzenverträglichkeit und geringer Warmblütertoxizität auch ausgezeichnete Wirksamkeit als Schädlingsbekämpfungsmittel aufweisen. Sie eignen sich vor allem zur Bekämpfung von Pflanzen und Tiere befallenden Schädlingen, insbesondere von Insekten.

Die erfindungsgemäss verwendeten und vorgeschlagenen Verbindungen der Formel I können in an sich bekannter Weise [vgl. J. Chem. Soc., Perkin Trans., 1 (2), 345-355 (1981); K.N. Zelenin et al., Khim. Geterotsikl. Soedin., No. 7, 904-910 (1982)] dadurch hergestellt werden, dass man eine Verbindung der Formel II

(II)

oxydiert und die erhaltene Verbindung der Formel I dann gegebenenfalls in herkömmlicher Weise in eines ihrer Salze umwandelt. Dieses Verfahren ist bevorzugt dadurch gekennzeichnet, dass man als Oxydationsmittel Sauerstoff verwendet. Es ist zur Herstellung von Verbindungen der vorliegenden Formel I bisher noch nicht eingesetzt worden.

Die Verbindungen der Formel I kann man nach bereits bekannten Verfahren auch dadurch herstellen, dass man Dipyridyl-1,3,4-oxdiazole der Formel III

(III)

oder Diacylhydrazine der Formel IV

(IV)

mit Phosphorsulfiden, z.B. $P_2S_5$, vorzugsweise in Gegenwart einer tertiären Stickstoffbase, umsetzt [vgl. schweizerische Patentschriften 411.906 und 426.848; J. Am. Chem. Soc. 80, 5201 (1958); J. Het. Chem. 1919 (1981)]. Die obigen Ausgangsstoffe der Formeln II, III und IV sind bekannt oder können nach üblichen Arbeitsweisen erhalten werden.

Die Verbindungen der Formel I eignen sich insbesondere zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemäss vorgeschlagenen Verbindungen der Formel I entspricht einer Abtötungsrate (Mortalität) von mindestens 50-60 % der erwähnten Schädlinge.

Mit Hilfe der erfindungsgemäss verwendeten Verbindungen der Formel I können vor allem pflanzenschädigende Insekten, speziell pflanzenschäigende Insekten in Zier- und Nutzpflanzungen, insbesondere in Baumwollkulturen, Gemüsekulturen, Reiskulturen und Obstkulturen bekämpft werden. In diesem Zusammenhang ist hervorzuheben, dass die genannten Verbindungen sich sowohl durch eine stark ausgeprägte systemische als auch Kontakt-Wirkung gegen saugende Insekten, vor allem gegen Insekten der Familie Aphididae (wie z.B. Aphis fabae, Aphis craccivora und Myzus persicae), welche sich mit herkömmlichen Mitteln nur schwierig bekämpfen lassen, auszeichnen.

Die Verbindungen der Formel I zeichnen sich weiterhin durch eine gute Wirkung gegen larvale Insektenstadien und Nymphen, speziell von fressenden Schadinsekten, aus.

Die Verbindungen der Formel I eignen sich ferner zur Bekämpfung von Ektoparasiten, z.B. Lucilia sericata, sowie von Zecken an Haus-und Nutztieren, z.B. durch Tier-, Stall- und Weidebehandlung.

Die Wirkung der erfindungsgemäss verwendeten Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden als Schädlingsbekämpfungsmittel in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze von und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979;

Dr. Helmut Stache "Tenside Taschenbuch", Carl Hanser Verlag München/Wien 1981.

Die erfindungsgemässen pestiziden Zubereitungen enthalten in der Regel 0,1 bis 99 %, insbesondere 0,1 bis 95 %, eines Wirkstoffes der Formel I oder Kombinationen davon mit andern Insektiziden oder Akariziden, 1

bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen, wie z.B. 0,1 bis 1000 ppm.

Die erfindungsgemässen Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1:

a) Herstellung von 1-(Nicotinoyl)-2-(3-pyridyliden)-hydrazin:

Zu einer Lösung von 41,1 g Nicotinsäurehydrazid und 32,1 g Pyridin-3-carbaldehyd in 300 ml Aethanol werden bei Raumtemperatur fünf Tropfen Eisessig zugegeben. Das leicht exotherm reagierende Reaktionsgemisch wird während ca. 2 Stunden bei Raumtemperatur gerührt, das ausgefallene Produkt abfiltriert und mit ca. 100 ml Aethanol nachgewaschen. Nach dem Trocknen erhält man die Titelverbindung der Formel

```
    .        O              .
 .// \.——— C-NH-N=CH———./ \\.
 |    ||                  ||   |
 .\   /.                  .\  //.
   \\N/                     \N//
```

mit einem Schmelzpunkt von 213° - 216°C.

b) Herstellung von 1-Chlor-1,4-di-(3-pyridyl)-2,3-diazabutadien

Es wird eine Suspension von 60,2 g des nach a) erhaltenen 1-(Nicotinoyl)-2-(3-pyridyliden)-hydrazins in 1100 ml Toluol am Rückfluss erhitzt. Zu dieser Suspension werden 94,9 g Thionylchlorid langsam hinzugetropft. Nach ca. achtstündigem Erhitzen am Rückfluss wird das Reaktionsgemisch mit einem Rotationsverdampfer eingeengt, der Rückstand in 700 ml Tetrahydrofuran gelöst und mit 41 g Triäthylamin behandelt. Nach ca. 30-minütigem Rühren bei Raumtemperatur wird das Reaktionsgemisch am Rotationsverdampfer eingeengt und der Rückstand in Essigester gelöst. Die erhaltene Lösung wird einmal mit Wasser sowie einmal mit gesättigter Kochsalzlösung gewaschen, über $Na_2SO_4$ getrocknet und eingedampft. Das zurückbleibende Rohprodukt wird mit heissem Tetrahydrofuran verrieben, abfiltriert und getrocknet. Man erhält so die Titelverbindung der Formel

```
    .       Cl            .
 .// \.——— C=N-N=CH———./ \\.
 |    ||                ||   |
 .\   /.                .\  //.
   \\N/                   \N//
```

mit einem Schmelzpunkt von 103° - 105°C.

c) Herstellung von Bis-2,5-(3-pyridyl)-4,5-dihydro-1,3,4-thiadiazol

Eine Lösung von 6,7 g 85%igem Kaliumhydroxyd in 190 ml Aethanol wird mit Schwefelwasserstoff gesättigt. Die so erhaltene Lösung wird mit 24,3 g des nach b) erhaltenen 1-Chlor-1,4-di-(3-pyridyl)-2,3-diazabutadiens portionsweise unter Eiskühlung versetzt. Das Reaktionsgemisch wird während 2 Stunden bei Raumtemperatur nachgerührt, eingeengt und in Essigester aufgenommen. Die Essigester-Lösung wird dann einmal mit Wasser sowie einmal mit gesättigter Kochsalzlösung gewaschen, über Natriumsulfat getrocknet, filtriert und eingedampft. Der Rückstand wird mit einem Hexan-Aether-Gemisch gewaschen und getrocknet. Man erhält auf diese Weise die Titelverbindung der Formel

```
           HN———N
            |   ||
    .      .     .      .
 .// \.  /  \S/  \  ./ \\.
 |    ||              ||   |
 .\   /.              .\  //.
   \\N/                 \N//
```

mit einem Schmelzpunkt von 77° - 78°C.

d) Herstellung von Bis-2,5-(3-pyridyl)-1,3,4-thiadiazol:

Durch eine auf Rückflusstemperatur erhitzte Lösung von 6 g des nach c) erhaltenen Bis-2,5-(3-pyridyl)-4,5-dihydro-1,3,4-thiadiazols in 70 ml Aethanol wird während ca. 20 Stunden ein langsamer Sauerstoff-Strom hindurchgeleitet. Nach dem Abkühlen des Ansatzes vom ausgefallenen Niederschlag abfiltriert, der mit wenig Aethanol nachgewaschen wird. Das erhaltene Rohprodukt wird aus Dioxan:Aethanol:Wasser (1:1:1) umkristallisiert und im Vakuum gerocknet. Man erhält die Titelverbindung der Formel

mit einem Schmelzpunkt von 219° - 219,2°C (Verbindung Nr. 1), bekannt aus der schweizerischen Patentschrift 411.906.

In üblicher und an sich bekannter Weise (Base + Säure = Salz + Wasser) wird von der erhaltenen Verbindung Nr. 1 auch das Oxalat (HOOC-COOH) vom Smp. 222-225°C sowie das Hydrochlorid (2 HCl) vom Smp. 256-260°C hergestellt.

Wie vorstehend angegeben werden auch die folgenden Verbindungen der Formel I hergestellt:

| Verbindung | physikalische Daten |
|---|---|
| 2 | Smp. 165-166°C |
| 3 | Smp. 204-205°C |
| 4 | Smp. 216-217°C |
| 5 | Smp. 236,5 - 238°C (bekannt aus der schweizerischen Patentschrift 411,906) |
| 6 | Smp. 201,3 - 202,7°C (bekannt aus der schweizerischen Patentschrift 411,906) |

Beispiel 2:

Formulierungen für Wirkstoffe der Formel I resp. Kombinationen dieser Wirkstoffe mit andern Insektiziden oder Akariziden (% = Gewichtsprozent):

| 1. Spritzpulver | a) | b) | c) |
|---|---|---|---|
| Wirkstoff oder Wirkstoff-kombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

2. Emulsions-Konzentrat

Wirkstoff oder Wirkstoffkombination 10 %
Octylphenolpolyäthylenglykoläther (4-5 Mol AeO) 3 %
Ca-Dodecylbenzolsulfonat 3 %
Ricinusölpolyglykoläther (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

4. Extruder-Granulat

Wirkstoff oder Wirkstoffkombination 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

Wirkstoff oder Wirkstoffkombination 3 %
Polyäthylenglykol (MG 200) 3 %
Kaolin 94 %

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

Wirkstoff oder Wirkstoffkombination 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Wirkung gegen Musca domestica

Je 50 g frisch zubereitetes CSMA-Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I zeigen gute Wirkung im obigen Test.

Beispiel 4: Wirkung gegen Aedes aegypti (Larven)

Auf die Oberfläche von 150 ml Wasser, das sich in einem Behälter befindet, wird so viel einer 0,1%igen acetonischen Lösung des Wirkstoffes pipettiert, dass eine Konzentration von 400 ppm erhalten wird. Nach Verdunsten des Acetons wird der Behälter mit 30 bis 40 2-tägigen Aedes-Larven beschickt. Nach 2 und 7 Tagen wird die Mortalität geprüft.

Verbindungen der Formel I zeigen gute Wirkung im obigen Test.

Beispiel 5: Insektizide Kontaktwirkung gegen Aphis craccivora

In Töpfen (20 ml Inhalt) angezogene 4-5 Tage alte Erbsenkeimlinge werden vor Versuchsbeginn mit je ca. 200 Individuen von Aphis craccivora besiedelt. Die so behandelten Pflanzen werden 24 Stunden später mit einer wässrigen Zubereitung enthaltend 400 ppm der zu prüfenden Verbindung bis zur Tropfnässe besprüht. Man verwendet pro Test-Verbindung zwei Pflanzen. Bonitur auf Mortalität der Test-Insekten erfolgt nach weiteren 3 und 5 Tagen. Der Versuch wird bei 21-22°C und einer relativen Luftfeuchtigkeit von etwa 60 % durchgeführt.

Verbindungen der Formel I zeigen gute Wirkung in diesem Test.

Beispiel 6: Insektizide systemische Wirkung gegen Aphis craccivora (Erde)

Es werden 4-5 Tage alte Erbsenkeimlinge (etwa 2 cm hoch) in Töpfen (12 cm Durchmesser), welche 600 ccm Erde enthalten, mit Aphis craccivora infestiert (etwa 200 Blattläuse pro Topf). In jedem Topf befinden sich 4 Keimlinge, auf denen sich die Blattlaus-Populationen entwickeln. Nach 24 Stunden giesst man 50 ml einer wässrigen Emulsions-Zubereitung der zu prüfenden Verbindungen (erhalten aus einem 25%igen Spritzpulver) in einer Konzentration von 400 ppm direkt auf die in den Töpfen befindliche Erde.

Die Auswertung der erzielten Abtötung der Test-Insekten erfolgt 2 und 3 Tage nach Wirkstoffapplikation. Pro Testsubstanz werden vier Pflanzen, jeweils in einem separaten Topf, verwendet. Der Versuch wird bei 25°C und ca. 70 % relativer Luftfeuchtigkeit durchgeführt.

Die Verbindungen der Formel I zeigen gute Wirkung in obigem Test.

Beispiel 7: Kontaktwirkung auf Myzus persicae

Etwa 4 bis 5 Tage alte, in Wasser angezogene Erbsenkeimlinge (Vicia faba) werden vor Versuchsbeginn jeweils mit ca. 200 Individuen der Spezies Myzus persicae besiedelt. Die so behandelten Pflanzen werden 24 Stunden später mit einer wässrigen Suspension enthaltend 12,5 ppm der zu prüfenden Verbindung bis zur Tropfnässe direkt besprüht. Man verwendet pro Testsubstanz zwei Pflanzen. Eine Auswertung der erzielten Abtötungsrate erfolgt 24, 48 und 72 Stunden nach Wirkstoff-Applikation. Der Versuch wird bei 21-22°C und etwa 60 % relativer Luftfeuchtigkeit durchgeführt.

Verbindung Nr. 1 gemäss Beispiel 1 ergibt eine 80-100%ige Mortalität in diesem Test.

Beispiel 8: Systemische Wirkung auf Myzus persicae (Erde)

Bewurzelte Paprikapflanzen in 4- bis 5-Blatt-Stadium werden in Töpfe, welche 60 ccm Erde enthalten, verpflanzt. Die Pflanzen werden dann jeweils mit 200 Individuen von Myzus persicae besiedelt. Anschliessend werden 20 ml einer wässrigen Formulierung der zu prüfenden Verbindung (erhalten aus einem 25%igen emulgierbaren Spritzpulver) jeweils in einer Konzentration von 400 ppm direkt auf die in den Töpfen befindliche Erde aufgegossen.

Die Auswertung der erzielten %-Abtötung erfolgt 3 und 7 Tage nach Wirkstoff-Applikation. Pro Testsubstanz werden zwei Pflanzen, je eine jeweils in separaten Töpfen, verwendet. Der Versuch wird bei ca. 22°C und 60 % relativer Luftfeuchtigkeit durchgeführt.

Verbindungen der Formel I zeigen gute Wirkung in obigem Test.

Beispiel 9: Systemische Wirkung auf Myzus persicae (Wasser)

Erbsenkeimlinge (ca. 2 cm hoch), die 24 Stunden vor Beginn des Versuches mit ca. 200 Blattläusen infestiert worden waren, werden in 20 ml einer wässrigen Emulsions-Zubereitung gestellt, die 100 ppm des zu prüfenden Wirkstoffes enthält. Die wässrige Zubereitung wird aus einem Emulsionskonzentrat oder einer benetzbaren Pulverformulierung des betreffenden Wirkstoffes hergestellt und befindet sich in einem Gefäss (Inhalt: 150 ml), dass mit einem Löcher aufweisenden Plastikdeckel abgeschlossen ist. Die Wurzel der infestierten Erbsenpflanze wird durch das zentrale Loch in dem Plastikdeckel in die wässrige Wirkstoff-Zubereitung geschoben. Dieses Loch wird dann mit Watte abgedichtet, um die Pflanze zu fixieren.

Der Versuch wird bei 21°C und 60 % relativer Luftfeuchtigkeit durchgeführt. Nach zwei, drei und sechs Tagen wird auf die Anzahl der nicht mehr saugfähigen Testtiere, im Vergleich zu unbehandelten Kontrollen, bonitiert. Damit wird festgestellt, ob der über die Wurzel aufgenommene Wirkstoff, die Blattläuse an den oberen Pflanzenteilen abtötet.

Verbindung Nr. 1 gemäss Beispiel 1 zeigt im obigen Versuch eine 80 - 100%ige systemische Wirkung gegen Insekten der Spezies Myzus persicae.

Beispiel 10: Blattpenetrations-Wirkung auf Aphis craccivora

In etwa 8 cm hohe Kunststoffbecher (Durchmesser etwa 6 cm) wird ein passender kleiner Zweig von Vicia faba (Pferdebohne) gelegt, der mit Blattläusen der Spezies Aphis craccivora stark infiziert ist. Der Becher wird mit einem Kunststoffdeckel, der in der Mitte eine ausgestanzte Oeffnung von 2 cm Durchmesser aufweist, bedeckt. Auf die in dem Deckel befindliche Oeffnung wird ein Blatt einer Vicia faba-Pflanze gelegt, ohne dieses Blatt von der eingetopften Pflanze abzutrennen. Das Blatt wird dann mit einem zweiten Lochdeckel auf dem Becher über der Oeffnung des ersten Deckels fixiert. Von der Unterseite her, d.h. durch die Oeffnung des ersten Deckels hindurch, infizieren nun die in dem Becher befindlichen Blattläuse das obenliegende Blatt der Futterpflanze. Auf der Oberseite wird auf das Blatt eine wässrige Zubereitung des zu prüfenden Wirkstoffes in einer Konzentration von 100 ppm mittels eines Pinsels gleichmässig aufgetragen. Es wird geprüft, ob die einseitig auf die Oberseite des Blattes der Futterpflanze aufgetragene Testsubstanz in genügender Menge durch das Blatt hindurch auf dessen Unterseite diffundiert, um dort saugende Blattläuse abzutöten.

Der Versuch wird bei etwa 21°C und 60 % relativer Luftfeuchtigkeit durchgeführt. Die Auswertung auf %-Mortalität erfolgt 24 und 48 Stunden nach Wirkstoffapplikation.

Verbindung Nr. 1 gemäss Beispiel 1 zeigt 80 - 100%ige Wirkung in obigem Test.

**Patentansprüche**

1. Verwendung einer Verbindung der Formel I

(I),

sowie der Salze einer Verbindung der Formel I zur Bekämpfung von Schädlingen.

2. Verwendung gemäss Anspruch 1 einer Verbindung der Formel Ia

(Ia).

3. Verwendung gemäss Anpruch 2 der Verbindung der Formel

.

4. Verwendung gemäss Anspruch 2 der Verbindung der Formel

.

5. Verwendung gemäss Anspruch 2 der Verbindung der Formel

.

6. Verwendung gemäss einem der Ansprüche 1 bis 5 zur Bekämpfung von Insekten und Vertretern der Ordnung Akarina an Tieren und Pflanzen.

7. Verwendung gemäss Anspruch 6 zur Bekämpfung von pflanzenschädigenden Insekten.

8. Verwendung gemäss Anspruch 7 zur Bekämpfung von pflanzenschädigenden saugenden Insekten.

9. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung der Formel I gemäss einem der Ansprüche 1 bis 5, einem Salz einer solchen Verbindung oder einem Mittel enthaltend neben Zusatz- und Trägerstoffen eine pestizid wirksame Menge dieser Verbindung oder eines Salzes derselben in Kontakt bringt oder behandelt.

10. Schädlingsbekämpfungsmittel, welches als aktive Komponente eine Verbindung der Formel I gemäss einem der Ansprüche 1 bis 5 oder ein Salz einer solchen Verbindung zusammen mit geeigneten Trägern und/oder anderen Zuschlagstoffen enthält.

11. Pflanzenphysiologisch unbedenkliches Salz einer Verbindung der Formel I gemäss Anspruch 1.

12. Verbindung der Formel

13. Verbindung der Formel

Europäisches Patentamt

# EUROPÄISCHER RECHERCHENBERICHT

Nummer der Anmeldung

EP 88810214.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | DD - A - 157 664 (SCHUSTER et al.)<br>* Anspruch 1; Seite 9, Zeile 16 *<br>-- | 1-5 | A 01 N 43/82<br>C 07 D 417/14 |
| D,A | EP - A1 - 0 116 515 (CIBA-GEIGY AG)<br>* Zusammenfassung *<br>-- | 1-10 | |
| D,A | CH - A - 411 906 (CIBA AKTIENGESELLSCHAFT)<br>* Seite 6, Verbindung Nr. 26; Seite 7, Verbindung Nr. 41; Seite 8, Verbindung Nr. 52 *<br>-- | 12,13 | |
| A | CHEMICAL ABSTRACTS, Band 48, Nr. 19, 10. Oktober 1954, Columbus, Ohio, USA<br>F.H.MCMILLAN et al. "Antitubercular substances. II. Substitution products of isonicotinic hydrazide"<br>Zusammenfassung-Nr. 11 413h<br>& J.Am.Pharm.Assoc. 42, 457-64 (1953)<br>-- | | **RECHERCHIERTE SACHGEBIETE (Int. Cl 4)**<br>A 01 N 43/00<br>C 07 D 417/00 |
| A | CHEMICAL ABSTRACTS, Band 49, Nr. 14, 25. Juli 1955, Columbus, Ohio, USA<br>H.B.KÖNIG et al. "Sulfur-containing derivatives of pyridinecarboxylic acids and compounds derived therefrom"<br>Zusammenfassung-Nr. 9 631d<br>& Chem.Ber. 87, 825-34 (1954)<br>-- | | |

Der vorliegende Recherchenbericht wurde für alle Patentansprüche erstellt.

| Recherchenort | Abschlußdatum der Recherche | Prüfer |
|---|---|---|
| WIEN | 17-06-1988 | SCHNASS |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze

- E : älteres Patentdokument, das jedoch erst am oder nach dem Anmeldedatum veröffentlicht worden ist
- D : in der Anmeldung angeführtes Dokument
- L : aus andern Gründen angeführtes Dokument

- & : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-

EP 88810214.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 54, Nr. 11, 10. Juni 1960, Columbus, Ohio, USA<br>A.WOLF et al. "The action of some central sedative compounds" Zusammenfassung-Nr. 11 289f<br>& Arzneimittel-Forsch. 10, 50-2 (1960)<br>-- | | |
| A | CHEMICAL ABSTRACTS, Band 101, Nr. 13, 24. September 1984, Columbus, Ohio, USA<br>S.KAMIYA et al. "Synthesis of anti-tumor heterocyclic compounds" Seite 643, Spalte 1, Zusammenfassung-Nr. 110 770n<br>& Eisei Shikensho Hokoku 1983, (101), 71-5<br>---- | | RECHERCHIERTE SACHGEBIETE (Int Cl 4) |

Der vorliegende Recherchenbericht wurde für alle Patentansprüche erstellt.

| Recherchenort | Abschlußdatum der Recherche | Prüfer |
|---|---|---|
| WIEN | 17-06-1988 | SCHNASS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82